# EUROPEAN PATENT SPECIFICATION

(11) **EP 4 231 959 B1**
(45) Date of publication and mention of the grant of the patent: **18.09.2024**
(21) Application number: 21820767.8
(22) Date of filing: 11.11.2021
(51) Int. Cl.: A61B 90/70

(54) **MEDICAL SYSTEMS FOR ULTRASONIC DECOMPOSITION OF INTRALUMINAL CLOTS**
MEDIZINISCHE SYSTEME ZUR ULTRASCHALLZERSETZUNG INTRALUMINALER GERINNSEL
SYSTÈMES MÉDICAUX POUR LA DÉCOMPOSITION ULTRASONORE DE CAILLOTS INTRALUMINAUX

(30) Priority: 12.11.2020 US 202063113074 P
(43) Date of publication of application: 30.08.2023
(73) Proprietor: Bard Access Systems, Inc., Salt Lake City, UT 84116 (US)
(72) Inventor: MCLAUGHLIN, William, Robert, Bountiful, UT 84010 (US); SOWARDS, Steffan, Salt Lake City, UT 84124 (US); ANDERSON, Devan, Taylorsville, UT 84123 (US); MISENER, Anthony, K., Bountiful, UT 84010 (US)
(74) Representative: Hoffmann Eitle
(86) International application number: PCT/US2021/058993
(87) International publication number: WO 2022/103970

(56) References cited:
- WO-A2-2018/014021
- CN-A- 108 283 758
- US-A- 6 001 069
- US-A1- 2002 069 893
- US-A1- 2018 168 668

## Description

### BACKGROUND

Thromboses, otherwise known as blood clots, can occur in blood vessels in which catheters such as peripheral intravenous catheters ("PIVCs"), peripherally inserted central catheters ("PICCs"), central venous catheters ("CVCs"), or the like are commonly placed. Like thromboses in blood vessels, intraluminal clots can occur in lumens of such catheters. The intraluminal clots are commonly treated with thrombolytic drugs such as alteplase, which is tissue plasminogen activator ("TPA") produced by recombinant DNA technology. TPA catalyzes conversion of an open form of clot-bound plasminogen into active plasmin, which is a major enzyme responsible for breaking down fibrin in thromboses.

In treating an intraluminal clot to restore patency to a catheter, a clinician typically forces a solution of TPA into a lumen of the catheter proximal of the intraluminal clot using a 3-way stopcock method. The method requires a drawing step of drawing fluid from the lumen of the catheter proximal of the clot with a first syringe, thereby creating a partial vacuum, an injecting step of injecting the solution of TPA into the lumen of the catheter under the vacuum with a second syringe, and a waiting step of waiting up to at least 20 minutes for the TPA to act on the intraluminal clot before repeating the foregoing steps. While the 3-way stopcock method can be effective, it can also take over an hour or more to treat an intraluminal clot due to its size, extent of occlusion, and location in the catheter. US 2018/168668A1 relates to an apparatus for use in a vascular space in a body. US 6 001 069 A relates to a catheter for use in a vessel of a body. CN 108 283 758 A relates to a drug-eluting balloon catheter with the functions of ultrasonic thrombus-breaking drug thrombolysis, thrombus suction and angioplasty. US 2002/069893 A1 relates to a cleaning apparatus for cleaning the interior surface of a channel of a medical instrument including an ultrasonic cleaning device having a cleaning catheter provided with ultrasonic vibrators for separating undesired matter from the interior surface of the channel of the medical instrument by ultrasonic vibration and an ultrasonic oscillator for operating the ultrasonic vibrators.

Disclosed herein are medical systems and methods thereof for ultrasonic decomposition of intraluminal clots that improve upon at least the 3-way stopcock method.

### SUMMARY

The invention is related to medical systems for ultrasonic decomposition of intraluminal clots as defined in the independent claims 1 and 8. Further embodiments are defined in the dependent claims.

Disclosed herein is a medical system for ultrasonic decomposition of intraluminal clots. The medical system includes, in some embodiments, a stylet. The stylet is configured to insert into a lumen of a catheter. The stylet includes one or more electrical impedance sensors and one or more ultrasound transducers. The one-or-more impedance sensors are in a distal portion of the stylet. The one-or more impedance sensors are configured to detect changes in impedance for identifying intraluminal clots in the catheter. The one-or-more ultrasound transducers are embedded in the distal portion of the stylet. The one-or-more ultrasound transducers are configured for decomposing the intraluminal clots to reestablish patency in the catheter.

In some embodiments, the one-or-more ultrasound transducers form an ultrasound-transducer array embedded along a length of stylet.

In some embodiments, the one-or-more ultrasound transducers form an ultrasound-transducer array embedded around a circumference of the stylet.

In some embodiments, the one-or-more ultrasound transducers are configured for decomposing the intraluminal clots by ultrasonic cavitation of fluid proximate of the intraluminal clots.

In some embodiments, the one-or-more ultrasound transducers are further configured for decomposing the intraluminal clots by ultrasonic agitation of a thrombolytic drug in the fluid proximate the intraluminal clots.

In some embodiments, the one-or-more ultrasound transducers are configured for decomposing the intraluminal clots by direct contact of the intraluminal clots with the stylet while the one-or-more ultrasound transducers are in operation.

In some embodiments, the one-or-more ultrasound transducers are configured to automatically activate upon identification of the intraluminal clots by the changes in impedance in accordance with logic onboard a console to which the stylet is connected in an operable state of the medical system.

Also disclosed herein is another medical system for ultrasonic decomposition of intraluminal clots. The medical system includes, in some embodiments, a stylet and an ultrasound probe. The stylet is configured to insert into a lumen of a catheter. The stylet includes one or more electrical impedance sensors and a resonant section of the stylet. The one-or-more impedance sensors are in a distal portion of the stylet. The one-or-more impedance-sensors are configured to detect changes in impedance for identifying intraluminal clots in the catheter. The resonant section of the stylet is in the distal portion of the stylet distal of the one-or-more impedance sensors. The resonant section of the stylet is configured to resonate with an externally applied ultrasonic frequency for decomposing the intraluminal clots to reestablish patency in the catheter. The ultrasound probe is configured to apply the ultrasonic frequency to the resonant section of the stylet.

In some embodiments, the resonant section of the stylet is configured for decomposing the intraluminal clots by ultrasonic cavitation of fluid proximate of the intraluminal clots when the ultrasonic frequency is applied thereto by the ultrasound probe.

In some embodiments, the resonant section of the stylet is further configured for decomposing the intraluminal clots by ultrasonic agitation of a thrombolytic drug in the fluid proximate the intraluminal clots when the ultrasonic frequency is applied thereto by the ultrasound probe.

In some embodiments, the resonant section of the stylet is configured for decomposing the intraluminal clots by direct contact of the intraluminal clots with the stylet when the ultrasonic frequency is applied thereto by the ultrasound probe.

In some embodiments, the ultrasound probe is configured to automatically activate upon identification of the intraluminal clots by the changes in impedance in accordance with logic onboard a console to which the stylet and ultrasound probe are functionally connected in an operable state of the medical system.

In some embodiments, the ultrasound probe is further configured for ultrasound imaging of the intraluminal clots for characterization thereof.

In some embodiments, the ultrasound probe is further configured for ultrasound imaging of the intraluminal clots for confirming the patency in the catheter after the reestablishing thereof.

Also disclosed herein is a method of medical system for ultrasonic decomposition of intraluminal clots. The method includes a stylet-inserting step, a clot-identifying step, and a clot-decomposing step. The stylet-inserting step includes inserting the stylet into a lumen of a catheter. The clot-identifying step includes identifying an intraluminal clot in the catheter by detecting changes in impedance with one or more electrical impedance sensors in a distal portion of the stylet. The clot-decomposing step includes decomposing the intraluminal clot with ultrasound, thereby reestablishing patency in the catheter.

In some embodiments, the clot-decomposing step further includes decomposing the intraluminal clot by ultrasonic cavitation of fluid proximate of the intraluminal clot.

In some embodiments, the clot-decomposing step further includes decomposing the intraluminal clot by ultrasonic agitation of a thrombolytic drug in the fluid proximate the intraluminal clot.

In some embodiments, the method further includes a drug-injecting step. The drug-injecting step includes injecting the thrombolytic drug into the lumen of the catheter before ultrasonic agitation of the thrombolytic drug in the fluid proximate the intraluminal clot in the clot-decomposing step.

In some embodiments, the clot-decomposing step further includes decomposing the intraluminal clot by direct contact of the intraluminal clot with the stylet while one-or-more ultrasound transducers are in operation.

In some embodiments, decomposing the intraluminal clot with ultrasound includes using ultrasound provided by the one-or-more ultrasound transducers formed into an array of ultrasound transducers embedded in the distal portion of the stylet.

In some embodiments, decomposing the intraluminal clot with ultrasound includes applying an ultrasonic frequency to a resonant section in the distal portion of the stylet with an ultrasound probe.

These and other features of the concepts provided herein will become more apparent to those of skill in the art in view of the accompanying drawings and following description, which describe particular embodiments of such concepts in greater detail.

### DRAWINGS

FIG. 1 illustrates a medical system for ultrasonic decomposition of intraluminal clots in accordance with some embodiments.
FIG. 2 illustrates a distal portion of a stylet of the medical system including an ultrasound-transducer array in accordance with some embodiments.
FIG. 3 illustrates the distal portion of another stylet of the medical system including another ultrasound-transducer array in accordance with some embodiments.
FIG. 4 illustrates the distal portion of yet another stylet of the medical system including a resonant section of the stylet in accordance with some embodiments.
FIG. 5 illustrates decomposing an intraluminal clot to reestablish patency in a catheter with the ultrasound-transducer array of the stylet of FIG. 2 in accordance with some embodiments.
FIG. 6 illustrates decomposing an intraluminal clot to reestablish patency in a catheter with the resonant section of the stylet of FIG. 4 in accordance with some embodiments.
FIG. 7 illustrates a block diagram of the medical system in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

As set forth above, intraluminal clots can occur in lumens of catheters such as PIVCs, PICCs, CVCs, or the like, and such intraluminal clots are commonly treated with thrombolytic drugs such as alteplase using a 3-way stopcock method. While the 3-way stopcock method can be effective, it can also take over an hour or more to treat an intraluminal clot due to its size, extent of occlusion, and location in the catheter. Disclosed herein are medical systems and methods thereof for ultrasonic decomposition of intraluminal clots that improve upon at least the 3-way stopcock method.

### Medical systems

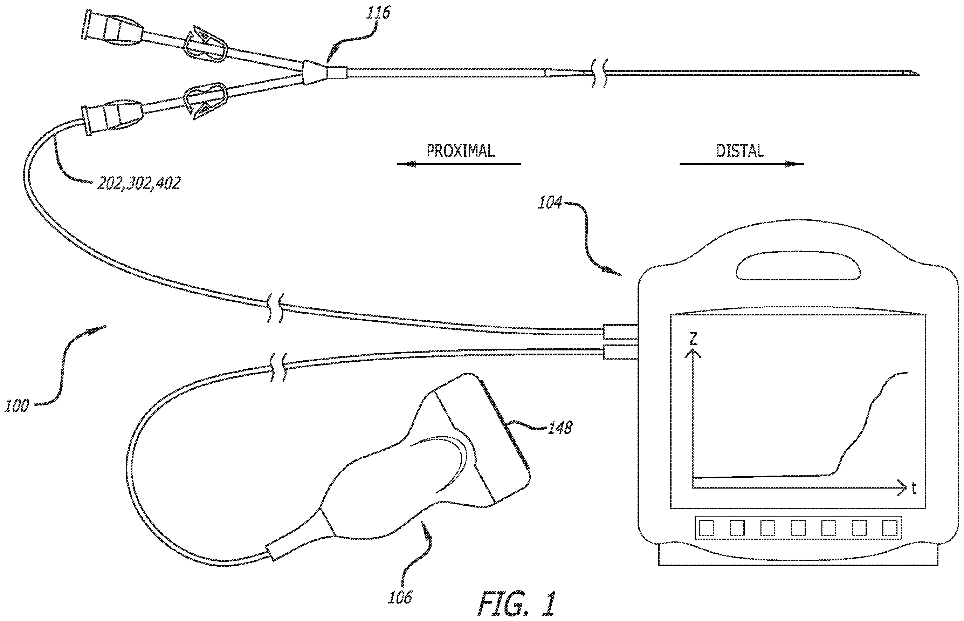

FIG. 1 illustrates a medical system 100 for ultrasonic decomposition of intraluminal clots in accordance with some embodiments.

As shown, the medical system 100 includes a stylet 202, 302, or 402 and, in some embodiments, a console 104 to which the stylet 202, 302, or 402 is connected in an operable state of the medical system 100. The medical system 100 can also include an ultrasound probe 106 functionally connected to the console 104 in an operable state of the medical system 100. Each of the foregoing components are described in more detail below beginning with the stylets 202, 302, and 402.

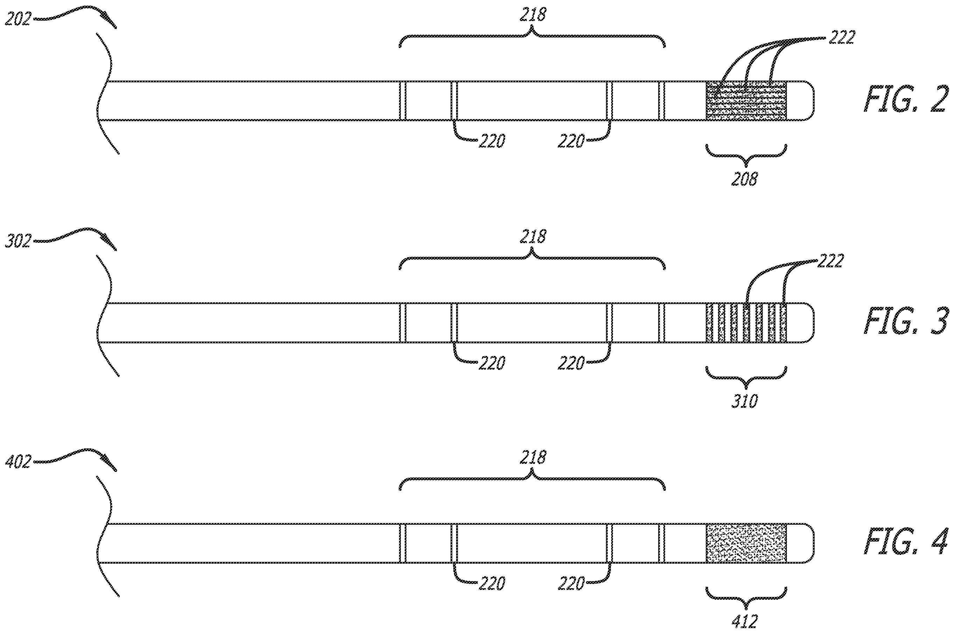

FIGS. 2-4 illustrate distal portions of the stylets 202, 302, and 402 of the medical system 100. FIG. 2 illustrates a distal portion of the stylet 202 of the medical system 100 including one or more ultrasound transducers 222 optionally in an ultrasound-transducer array 208 in accordance with some embodiments. FIG. 3 illustrates the distal portion of the stylet 302 of the medical system 100 including the one-or-more ultrasound transducers 222 optionally in another ultrasound-transducer array 310 in accordance with some embodiments. FIG. 4 illustrates the distal portion of the stylet 402 of the medical system 100 including a resonant section 412 of the stylet 402 in accordance with some embodiments.

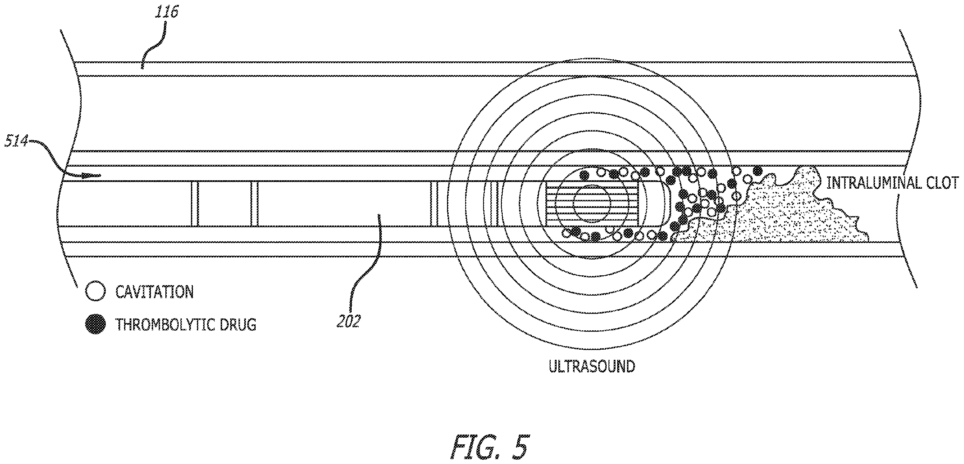

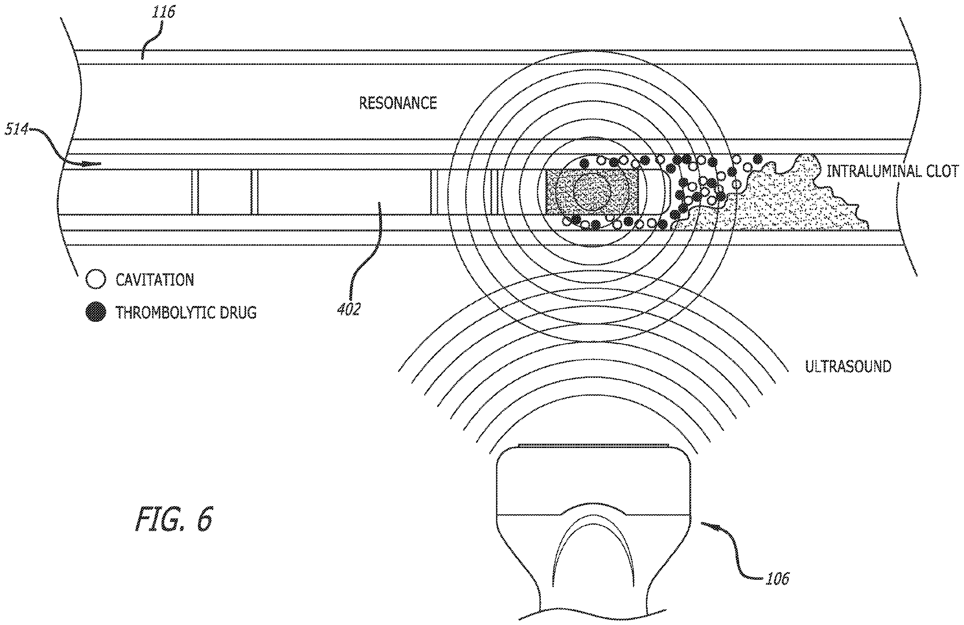

The stylet 202, 302, or 402 is configured to insert into a lumen 514 of a catheter 116 for identifying any intraluminal clots therein and, if present, ultrasonically decomposing the intraluminal clots. (*See* FIGS. 5 and 6.)

The stylet 202, 302, or 402 includes one or more electrical impedance sensors 220 such as ring electrodes optionally in an impedance-sensor array 218 in the distal portion of the stylet 202, 302, or 402. The one-or-more impedance sensors 220 are configured to detect changes in impedance for identifying any intraluminal clots in the catheter 116, and the one-or-more impedance sensors 220 can include any number of impedance sensors required for monopolar or multipolar (e.g., bipolar, tetrapolar, etc.) impedance measurements therefor.

The stylet 202 or 302 includes the one-or-more ultrasound transducers 222 optionally in the ultrasound-transducer array 208 or 310 embedded in the distal portion of the stylet 202 or 302 distal of the one-or-more impedance sensors 220 configured for decomposing any intraluminal clots in the catheter 116 and reestablishing patency thereof. FIG. 2 illustrates the one-or-more ultrasound transducers 222 formed into the ultrasound-transducer array 208 embedded along a length of the stylet 202, while FIG. 3 illustrates the one-or-more ultrasound transducers 222 formed into the ultrasound-transducer array 310 embedded around a circumference of the stylet 302. However, the one-or more ultrasound transducers 222 of either the stylet 202 or 302 can be in any arrangement required for decomposing any intraluminal clots in the catheter 116. Indeed, the one-or-more ultrasound transducers 222 can be in the ultrasound-transducer array 208 or 310 or another arrangement as required for decomposing any intraluminal clots in the catheter 116 by ultrasonic cavitation of fluid proximate the intraluminal clots, ultrasonic agitation of a thrombolytic drug (e.g., alteplase) in the fluid proximate the intraluminal clots, direct contact of the intraluminal clots with the stylet 202 or 302 while the one-or-more ultrasound transducers 222 are in operation, or a combination thereof.

As an alternative to the stylet 202 or 302 including the one-or-more ultrasound-transducers 222, the stylet 402 includes the resonant section 412 in the distal portion of the stylet 402 distal of the one-or-more impedance sensors 220 configured for decomposing any intraluminal clots in the catheter 116 and reestablishing patency thereof. The resonant section 412 of the stylet 402 is configured to resonate with an externally applied ultrasonic frequency for decomposing any intraluminal clots in the catheter 116. For example, the ultrasound probe 106 can be configured to apply the ultrasonic frequency to the resonant section 412 of the stylet 402. Indeed, the resonant section 412 of the stylet 402 can be configured for decomposing any intraluminal clots by ultrasonic cavitation of fluid proximate the intraluminal clots, ultrasonic agitation of a thrombolytic drug in the fluid proximate the intraluminal clots, direct contact of the intraluminal clots with the stylet 402, or the like when the ultrasonic frequency is applied to the resonant section 412 of the stylet 402 by the ultrasound probe 106.

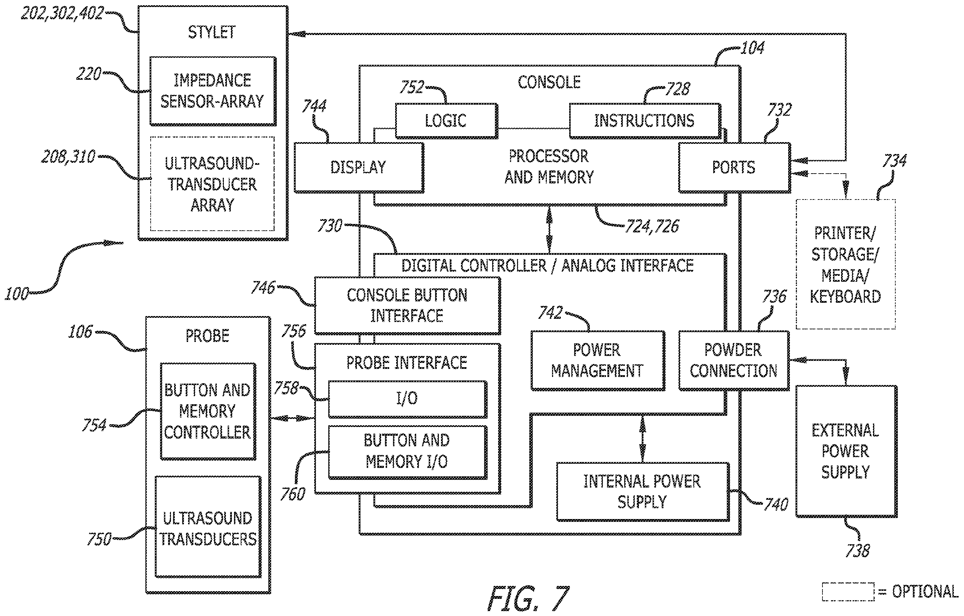

FIG. 7 illustrates a block diagram of the medical system in accordance with some embodiments.

As set forth above, the medical system 100 includes the stylet 202, 302, or 402, the optional ultrasound probe 106, and, in some embodiments, the console 104 to which the stylet 202, 302, or 402 and, when present, the ultrasound probe 106 are functionally connected in an operable state of the medical system 100. When the ultrasound probe 106 is present and functionally connected to the console 104, the medical system 100 is configured for ultrasound imaging of any intraluminal clots in the catheter 116 for characterization thereof. Advantageously, such a medical system can be used for confirming patency of the catheter 116 by ultrasound imaging after decomposing any intraluminal clots in the catheter 116 and reestablishing the patency thereof.

The console 104 houses and accommodates a variety of components of the medical system 100, and it is appreciated the console 104 can take any of a variety of forms. A processor 724 and memory 726 such as random-access memory ("RAM") or non-volatile memory (e.g., electrically erasable programmable read-only memory ["EEPROM"]) is included in the console 104 for controlling functions of the medical system 100, as well as executing various logic operations or algorithms during operation of the medical system 100 in accordance executable instructions 728 therefor stored in the memory 726 for execution by the processor 724. For example, the console 104 is configured to instantiate by way of the instructions 728 one or more processes for identifying or decomposing any intraluminal clots in the catheter 116, as well as process electrical signals from the ultrasound probe 106 into ultrasound images for the ultrasound imaging. A digital controller/analog interface 730 is also included with the console 104 and is in communication with both the processor 724 and other system components to govern interfacing between the ultrasound probe 106 and other system components set forth herein.

The console 104 further includes ports 732 for connection with additional components such as the stylet 202, 302, or 402 and optional components 734 including a printer, storage media, keyboard, etc. The ports 732 can be universal serial bus ("USB") ports, though other types of ports can be used for this connection or any other connections shown or described herein. A power connection 736 is included with the console 104 to enable operable connection to an external power supply 738. An internal power supply 740 (e.g., a battery) can also be employed either with or exclusive of the external power supply 738. Power management circuitry 742 is included with the digital controller/analog interface 730 of the console 104 to regulate power use and distribution.

A display screen 744 (e.g., a liquid-crystal display ["LCD"] screen) is integrated into the console 104 to provide a GUI and display information for a clinician during such as ultrasound images of intraluminal clots attained by the ultrasound probe 106. Alternatively, the display screen 744 is separate from the console 104 and communicatively coupled thereto. A console button interface 746 and control buttons included on the ultrasound probe 106 can be used to immediately call up a desired mode to the display screen 744 by the clinician for identifying or decomposing any intraluminal clots in the catheter 116.

The ultrasound probe 106 includes a probe head 148 that houses an array of ultrasound transducers 750, wherein the ultrasound transducers 750 are piezoelectric transducers or capacitive micromachined ultrasound transducers ("CMUTs"). The probe head 148 is configured for placement against skin of a patient over the catheter 116. In this way, the medical system 100, by way of the ultrasound probe 106 and logic 752, is able to characterize any intraluminal clots in the catheter 116 or confirm the patency of the catheter 116 by ultrasound imaging. Advantageously, the medical system 100 can be configured to automatically activate the one-or more ultrasound transducers 222 upon identification of any intraluminal clots by changes in impedance in accordance with the logic 752 onboard the console 104.

The ultrasound probe 106 also includes a button-and-memory controller 754 for governing button operation, as well as governing operation of the ultrasound probe 106. The button-and-memory controller 754 can include non-volatile memory (e.g., electrically erasable, programmable read-only memory ["EEPROM"]). The button-and-memory controller 754 is in operable communication with a probe interface 756 of the console 104, which includes an input/output ("VO") component 758 for interfacing with the ultrasound transducers 750 and a button-and-memory I/O component 760 for interfacing with the button-and-memory controller 754.

### Methods

FIG. 5 illustrates decomposing an intraluminal clot to reestablish patency in the catheter 116 with the one-or more ultrasound transducer 222 optionally in the ultrasound-transducer array 208 or 310 of the stylet 202 or 302 in accordance with some embodiments. FIG. 6 illustrates decomposing an intraluminal clot to reestablish patency in the catheter 116 with the resonant section 412 of the stylet 402 in accordance with some embodiments.

Methods of the medical systems set forth above include methods of using the medical systems. For example, a method of using the medical system 100 for ultrasonically decomposing intraluminal clots includes an optional stylet-connecting step, a stylet-inserting step, a clot-identifying step, and a clot-decomposing step.

The stylet-connecting step, when part of the method, includes functionally connecting the stylet 202, 302, or 402 to the console 104 as shown in FIG. 1.

The stylet-inserting step includes inserting the stylet 202, 302, or 402 into a lumen of a catheter such as the catheter 116 as shown in FIG. 5 for the stylet 202 and FIG. 6 for the stylet 402.

The clot-identifying step includes identifying an intraluminal clot in the catheter 116 by detecting changes in impedance with the one-or-more impedance sensors 220 in the distal portion of the stylet 202. 302, or 402.

The clot-decomposing step includes decomposing the intraluminal clot with ultrasound to reestablish patency in the catheter 116, which, in turn, can include decomposing the intraluminal clot by ultrasonic cavitation of fluid proximate the intraluminal clot, decomposing the intraluminal clot by ultrasonic agitation of a thrombolytic drug in the fluid proximate the intraluminal clot, decomposing the intraluminal clot by direct contact of the intraluminal clot with the stylet 202, 302, or 402 while the one-or-more ultrasound transducers 222 are in operation or the resonating section 412 of the stylet 402 is resonating with an ultrasonic frequency applied by the ultrasound probe 106, or a combination thereof.

The method can further include a drug-injecting step in association with the clot-decomposing step of decomposing the intraluminal clot by ultrasonically agitating the thrombolytic drug in the fluid proximate the intraluminal clot. The drug-injecting step includes injecting the thrombolytic drug into the lumen of the catheter 116 before ultrasonically agitating the thrombolytic drug in the fluid proximate the intraluminal clot in the clot-decomposing step.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the appended claims.

## Claims

1. A medical system (100) for ultrasonic decomposition of intraluminal clots, comprising:
a stylet (202, 302, 402) configured to insert into a lumen (514) of a catheter (116) including a fluid, the stylet (202, 302, 402) including:
one or more electrical impedance sensors (220) in a distal portion of the stylet (202, 302, 402), the one-or-more impedance sensors (220) configured to detect changes in impedance for identifying intraluminal clots in the catheter (116); and
one or more ultrasound transducers (222) embedded in the distal portion of the stylet (202, 302, 402), the one-or-more ultrasousnd transducers (222) configured for decomposing the intraluminal clots to reestablish patency in the catheter (116).

2. The medical system (100) of claim 1, wherein the one-or-more ultrasound transducers (222) form an ultrasound-transducer array (208) embedded along a length of stylet (202, 302, 402).

3. The medical system (100) of claim 1, wherein the one-or-more ultrasound transducers (222) form an ultrasound-transducer array (208) are embedded around a circumference of the stylet (202, 302, 402).

4. The medical system (100) of any claim of claims 1-3, wherein the one-or-more ultrasound transducers (222) are configured for decomposing the intraluminal clots by ultrasonic cavitation of the fluid proximate of the intraluminal clots.

5. The medical system (100) of any claim of claims 1-4, wherein the one-or-more ultrasound transducers (222) are configured for decomposing the intraluminal clots by ultrasonic agitation of a thrombolytic drug in the fluid proximate the intraluminal clots.

6. The medical system (100) of any claim of claims 1-5, wherein the one-or-more ultrasound transducers (222) are configured for decomposing the intraluminal clots by direct contact of the intraluminal clots with the stylet (202, 302, 402) while the one-or-more ultrasound transducers (222) are in operation.

7. The medical system (100) of any claim of claims 1-6, wherein the one-or-more ultrasound transducers (222) are configured to automatically activate upon identification of the intraluminal clots by the changes in impedance in accordance with logic onboard a console (104) to which the stylet (202, 302, 402) is functionally connected in an operable state of the medical system (100).

8. A medical system (100) for ultrasonic decomposition of intraluminal clots, comprising:
a stylet (202, 302, 402) configured to insert into a lumen (514) of a catheter (116) including a fluid, the stylet (202, 302, 402) including:
one or more electrical impedance sensors (220) in a distal portion of the stylet (202, 302, 402), the one-or-more impedance sensors (220) configured to detect changes in impedance for identifying intraluminal clots in the catheter (116); and
a resonant section of the stylet (202, 302, 402) in the distal portion of the stylet (202, 302, 402) distal of the one-or-more impedance sensors (220), the resonant section of the stylet (202, 302, 402) configured to resonate with an applied ultrasonic frequency for decomposing the intraluminal clots to reestablish patency in the catheter (116); and
an ultrasound probe (106) configured to apply the ultrasonic frequency to the resonant section of the stylet (202, 302, 402).

9. The medical system (100) of claim 8, wherein the resonant section of the stylet (202, 302, 402) is configured for decomposing the intraluminal clots by ultrasonic cavitation of the fluid proximate of the intraluminal clots when the ultrasonic frequency is applied thereto by the ultrasound probe (106).

10. The medical system (100) of either claim 8 or 9, wherein the resonant section of the stylet (202, 302, 402) is configured for decomposing the intraluminal clots by ultrasonic agitation of a thrombolytic drug in the fluid proximate the intraluminal clots when the ultrasonic frequency is applied thereto by the ultrasound probe (106).

11. The medical system (100) of any claim of claims 8-10, wherein the resonant section of the stylet (202, 302, 402) is configured for decomposing the intraluminal clots by direct contact of the intraluminal clots with the stylet (202, 302, 402) when the ultrasonic frequency is applied thereto by the ultrasound probe (106).

12. The medical system (100) of any claim of claims 8-11, wherein the ultrasound probe (106) is configured to automatically activate upon identification of the intraluminal clots by the changes in impedance in accordance with logic onboard a console to which the stylet (202, 302, 402) is connected in an operable state of the medical system (100).

13. The medical system (100) of any claim of claims 8-12, wherein the ultrasound probe (106) is further configured for ultrasound imaging of the intraluminal clots for characterization thereof.

14. The medical system (100) of any claim of claims 8-13, wherein the ultrasound probe (106) is further configured for ultrasound imaging of the intraluminal clots for confirming the patency in the catheter (116) after the reestablishing thereof.

## Patentansprüche

1. Medizinisches System (100) zur Ultraschallzersetzung intraluminaler Gerinnsel, umfassend:
ein Stilett (202, 302, 402), das so ausgebildet ist, dass es in ein Lumen (514) eines Katheters (116) eingeführt werden kann, einschließlich eines Fluids,
wobei das Stilett (202, 302, 402) einschließt:
einen oder mehrere elektrische Impedanzsensoren (220) in einem distalen Abschnitt des Stiletts (202, 302, 402), wobei der eine oder die mehreren Impedanzsensoren (220) so ausgebildet sind, dass sie Impedanzänderungen zur Identifizierung intraluminaler Gerinnsel in dem Katheter (116) detektieren; und
einen oder mehrere Ultraschallwandler (222), die in den distalen Abschnitt des Stiletts (202, 302, 402) eingebettet sind, wobei der eine oder die mehreren Ultraschallwandler (222) so ausgebildet sind, dass sie die intraluminalen Gerinnsel zersetzen, um die Durchgängigkeit in dem Katheter (116) wiederherzustellen.

2. Medizinisches System (100) nach Anspruch 1, wobei der eine oder die mehreren Ultraschallwandler (222) eine Ultraschallwandleranordnung (208) bilden, die entlang einer Länge des Stiletts (202, 302, 402) eingebettet ist.

3. Medizinisches System (100) nach Anspruch 1, wobei der eine oder die mehreren Ultraschallwandler (222) eine Ultraschallwandleranordnung (208) bilden, um einen Umfang des Stiletts (202, 302, 402) herum eingebettet sind.

4. Medizinisches System (100) nach einem der Ansprüche 1 bis 3, wobei der eine oder die mehreren Ultraschallwandler (222) so ausgebildet sind, dass sie die intraluminalen Gerinnsel durch Ultraschallkavitation des Fluids in der Nähe der intraluminalen Gerinnsel zersetzen.

5. Medizinisches System (100) nach einem der Ansprüche 1 bis 4, wobei der eine oder die mehreren Ultraschallwandler (222) so ausgebildet sind, dass sie die intraluminalen Gerinnsel durch Ultraschallbewegung eines thrombolytischen Medikaments in dem Fluid in der Nähe der intraluminalen Gerinnsel zersetzen.

6. Medizinisches System (100) nach einem der Ansprüche 1 bis 5, wobei der eine oder die mehreren Ultraschallwandler (222) so ausgebildet sind, dass sie die intraluminalen Gerinnsel durch direkten
Kontakt der intraluminalen Gerinnsel mit dem Stilett (202, 302, 402) zersetzen, während der eine oder die mehreren Ultraschallwandler (222) in Betrieb sind.

7. Medizinisches System (100) nach einem der Ansprüche 1 bis 6, wobei der eine oder die mehreren Ultraschallwandler (222) so ausgebildet sind, dass sie sich automatisch aktivieren, wenn die intraluminalen Gerinnsel durch die Impedanzänderungen in Übereinstimmung mit einer Logik an Bord einer Konsole (104) identifiziert werden, mit der das Stilett (202, 302, 402) in einem betriebsbereiten Zustand des medizinischen Systems (100) funktionell verbunden ist.

8. Medizinisches System (100) zur Ultraschallzersetzung intraluminaler Gerinnsel, umfassend:
ein Stilett (202, 302, 402), das so ausgebildet ist, dass es in ein Lumen (514) eines Katheters (116) eingeführt werden kann, einschließlich eines Fluids,
wobei das Stilett (202, 302, 402) einschließt:
einen oder mehrere elektrische Impedanzsensoren (220) in einem distalen Abschnitt des Stiletts (202, 302, 402), wobei der eine oder die mehreren Impedanzsensoren (220) so ausgebildet sind, dass sie Impedanzänderungen zur Identifizierung intraluminaler Gerinnsel in dem Katheter (116) detektieren; und
einen Resonanzabschnitt des Stiletts (202, 302, 402) in dem distalen Abschnitt des Stiletts (202, 302, 402) distal von dem einen oder den mehreren Impedanzsensoren (220), wobei der Resonanzabschnitt des Stiletts (202, 302, 402) so ausgebildet ist, dass er mit einer angelegten Ultraschallfrequenz in Resonanz tritt, um die intraluminalen Gerinnsel zu zersetzen, um die Durchgängigkeit in dem Katheter (116) wiederherzustellen; und
eine Ultraschallsonde (106), die so ausgebildet ist, dass sie die Ultraschallfrequenz an den Resonanzabschnitt des Stiletts (202, 302, 402) anlegt.

9. Medizinisches System (100) nach Anspruch 8, wobei der Resonanzabschnitt des Stiletts (202, 302, 402) so ausgebildet ist, dass er die intraluminalen Gerinnsel durch Ultraschallkavitation des Fluids in der Nähe der intraluminalen Gerinnsel zersetzt, wenn die Ultraschallfrequenz durch die Ultraschallsonde (106) daran angelegt wird.

10. Medizinisches System (100) nach Anspruch 8 oder 9, wobei der Resonanzabschnitt des Stiletts (202, 302, 402) so ausgebildet ist, dass er die intraluminalen Gerinnsel durch Ultraschallbewegung eines thrombolytischen Medikaments in dem Fluid in der Nähe der intraluminalen Gerinnsel zersetzt, wenn die Ultraschallfrequenz durch die Ultraschallsonde (106) daran angelegt wird.

11. Medizinisches System (100) nach einem der Ansprüche 8 bis 10, wobei der Resonanzabschnitt des Stiletts (202, 302, 402) so ausgebildet ist, dass er die intraluminalen Gerinnsel durch direkten Kontakt der intraluminalen Gerinnsel mit dem Stilett (202, 302, 402) zersetzt, wenn die Ultraschallfrequenz durch die Ultraschallsonde (106) daran angelegt wird.

12. Medizinisches System (100) nach einem der Ansprüche 8 bis 11, wobei die Ultraschallsonde (106) so ausgebildet ist, dass sie nach der Identifizierung der intraluminalen Gerinnsel durch die Impedanzänderungen in Übereinstimmung mit einer Logik an Bord einer Konsole automatisch aktiviert wird, mit der das Stilett (202, 302, 402) in einem betriebsbereiten Zustand des medizinischen Systems (100) verbunden ist.

13. Medizinisches System (100) nach einem der Ansprüche 8 bis 12, wobei die Ultraschallsonde (106) weiter für die Ultraschallbildgebung der intraluminalen Gerinnsel zu deren Charakterisierung ausgebildet ist.

14. Medizinisches System (100) nach einem der Ansprüche 8 bis 13, wobei die Ultraschallsonde (106) weiter für die Ultraschallbildgebung der intraluminalen Gerinnsel zur Bestätigung der Durchgängigkeit in dem Katheter (116) nach dessen Wiederherstellung ausgebildet ist.

## Revendications

1. Système médical (100) pour la décomposition ultrasonore de caillots intraluminaux, comprenant :
un stylet (202, 302, 402) configuré pour être inséré dans une lumière (514) d'un cathéter (116) incluant un fluide, le stylet (202, 302, 402) incluant :
un ou plusieurs capteurs (220) d'impédance électrique dans une partie distale du stylet (202, 302, 402), les un ou plusieurs capteurs (220) d'impédance étant configurés pour détecter des changements d'impédance pour identifier des caillots intraluminaux dans le cathéter (116) ; et
un ou plusieurs transducteurs ultrasonores (222) incorporés dans la partie distale du stylet (202, 302, 402), les un ou plusieurs transducteurs ultrasonores (222) étant configurés pour décomposer les caillots intraluminaux afin de rétablir la perméabilité dans le cathéter (116).

2. Système médical (100) selon la revendication 1, dans lequel les un ou plusieurs transducteurs ultrasonores (222) forment un réseau (208) de transducteurs ultrasonores incorporé sur une longueur de stylet (202, 302, 402).

3. Système médical (100) selon la revendication 1, dans lequel les un ou plusieurs transducteurs ultrasonores (222) qui forment un réseau (208) de transducteurs ultrasonores sont incorporés autour d'une circonférence du stylet (202, 302, 402).

4. Système médical (100) selon l'une quelconque des revendications 1 à 3, dans lequel les un ou plusieurs transducteurs ultrasonores (222) sont configurés pour décomposer les caillots intraluminaux par cavitation ultrasonore du fluide à proximité des caillots intraluminaux.

5. Système médical (100) selon l'une quelconque des revendications 1 à 4, dans lequel les un ou plusieurs transducteurs ultrasonores (222) sont configurés pour décomposer les caillots intraluminaux par agitation ultrasonore d'un médicament thrombolytique dans le fluide à proximité des caillots intraluminaux.

6. Système médical (100) selon l'une quelconque des revendications 1 à 5, dans lequel les un ou plusieurs transducteurs ultrasonores (222) sont configurés pour décomposer les caillots intraluminaux par contact direct des caillots intraluminaux avec le stylet (202, 302, 402) tandis que les un ou plusieurs transducteurs ultrasonores (222) sont en cours de fonctionnement.

7. Système médical (100) selon l'une quelconque des revendications 1 à 6, dans lequel les un ou plusieurs transducteurs ultrasonores (222) sont configurés pour s'activer automatiquement lors de l'identification des caillots intraluminaux par les changements d'impédance conformément à une logique embarquée sur une console (104) à laquelle le stylet (202, 302, 402) est fonctionnellement raccordé dans un état apte au fonctionnement du système médical (100).

8. Système médical (100) pour décomposition ultrasonore de caillots intraluminaux, comprenant :
un stylet (202, 302, 402) configuré pour être inséré dans une lumière (514) d'un cathéter (116) incluant un fluide, le stylet (202, 302, 402) incluant :
un ou plusieurs capteurs (220) d'impédance électrique dans une partie distale du stylet (202, 302, 402), les un ou plusieurs capteurs (220) d'impédance étant configurés pour détecter des changements d'impédance pour identifier des caillots intraluminaux dans le cathéter (116) ; et
une section résonante du stylet (202, 302, 402) dans la partie distale du stylet (202, 302, 402) distale des un ou plusieurs capteurs (220) d'impédance, la section résonante du stylet (202, 302, 402) étant configurée pour résonner avec une fréquence ultrasonore appliquée pour décomposer les caillots intraluminaux afin de rétablir la perméabilité dans le cathéter (116) ; et
une sonde ultrasonore (106) configurée pour appliquer la fréquence ultrasonore à la section résonante du stylet (202, 302, 402).

9. Système médical (100) selon la revendication 8, dans lequel la section résonante du stylet (202, 302, 402) est configurée pour décomposer les caillots intraluminaux par cavitation ultrasonore du fluide à proximité des caillots intraluminaux lorsque la fréquence ultrasonore est appliquée à ceux-ci par la sonde ultrasonore (106).

10. Système médical (100) selon la revendication 8 ou la revendication 9, dans lequel la section résonante du stylet (202, 302, 402) est configurée pour décomposer les caillots intraluminaux par agitation ultrasonore d'un médicament thrombolytique dans le fluide à proximité des caillots intraluminaux lorsque la fréquence ultrasonore est appliquée à ceux-ci par la sonde ultrasonore (106).

11. Système médical (100) selon l'une quelconque des revendications 8 à 10, dans lequel la section résonante du stylet (202, 302, 402) est configurée pour décomposer les caillots intraluminaux par contact direct des caillots intraluminaux avec le stylet (202, 302, 402) lorsque la fréquence ultrasonore est appliquée à ceux-ci par la sonde ultrasonore (106).

12. Système médical (100) selon l'une quelconque des revendications 8 à 11, dans lequel la sonde ultrasonore (106) est configurée pour s'activer automatiquement lors de l'identification des caillots intraluminaux par les changements d'impédance conformément à une logique embarquée sur une console à laquelle le stylet (202, 302, 402) est raccordé dans un état apte au fonctionnement du système médical (100).

13. Système médical (100) selon l'une quelconque des revendications 8 à 12, dans lequel la sonde ultrasonore (106) est en outre configurée pour imager par ultrasons les caillots intraluminaux en vue de la caractérisation de ceux-ci.

14. Système médical (100) selon l'une quelconque des revendications 8 à 13, dans lequel la sonde ultrasonore (106) est en outre configurée pour imager par ultrasons les caillots intraluminaux en vue de confirmer la perméabilité dans le cathéter (116) après le rétablissement de celle-ci.
